(19) **Europäisches Patentamt / European Patent Office / Office européen des brevets**

(11) **EP 4 481 385 A1**

(12) # EUROPEAN PATENT APPLICATION
published in accordance with Art. 153(4) EPC

(43) Date of publication:
**25.12.2024 Bulletin 2024/52**

(21) Application number: **23775114.4**

(22) Date of filing: **24.03.2023**

(51) International Patent Classification (IPC):
**G01N 33/531** $^{(2006.01)}$

(52) Cooperative Patent Classification (CPC):
**G01N 33/531**

(86) International application number:
**PCT/JP2023/012020**

(87) International publication number:
**WO 2023/182521 (28.09.2023 Gazette 2023/39)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **25.03.2022 JP 2022050801**

(71) Applicant: **Sekisui Medical Co., Ltd.
Tokyo 103-0027 (JP)**

(72) Inventor: **SAHARA, Ryo
Tokyo 103-0027 (JP)**

(74) Representative: **Maiwald GmbH
Elisenhof
Elisenstraße 3
80335 München (DE)**

(54) **IMMUNOASSAY REAGENT AND IMMUNOASSAY METHOD**

(57)    An immunoassay reagent, including: a substance that exhibits an immune reaction; and an insoluble carrier having a function to bind to the substance, the insoluble carrier including an insoluble carrier that is bound to the substance and an insoluble carrier that is not bound to the substance.

**EP 4 481 385 A1**

**Description**

Technical Field

The present disclosure relates to an immunoassay reagent and an immunoassay method.

Background Art

In an immunological analysis method for biological specimens, a substance that is immobilized at an insoluble carrier, such as particles, may be used.

For example, there is a method of binding a labeling substance such as a luminous material to a substance by immune reaction, wherein the substance is immobilized at an insoluble carrier included in a reagent, and detecting a signal emitted from the labeling substance.

When the reagent includes a substance that is immobilized at an insoluble carrier, a part of the substance may dissociate from the insoluble carrier depending on the storage conditions of the reagent, or the like. When dissociation of the substance from the insoluble carrier in the reagent progresses, the dissociated substance may adversely affect the immune reaction, or the amount of the substance immobilized at the insoluble carrier may change, thereby causing the measured values to fluctuate.

As a measure for improving the storage stability of a reagent including a substance immobilized at an insoluble carrier, Patent Document 1 describes an immunoassay reagent that includes an insoluble-carrier particulate reagent, in which an antibody or an antigen with respect to a target substance in a specimen is immobilized at insoluble-carrier particles, together with insoluble-carrier particles (dummy particles) that do not have specificity with respect to the insoluble-carrier particulate reagent or the target substance.

Prior Art Document

Patent Document

Patent Document 1: Japanese Patent Application Laid-Open (JP-A) No. 2000-321279

Summary of the Invention

Problem to be Solved by the Invention

Patent Document 1 describes that the coexistence of the insoluble-carrier particulate reagent and the dummy particles causes a shift in a balance of dissociation/adsorption toward the adsorption-side and suppresses the dissociation of the antibody or the antigen from the carrier particles. However, there may be a case in which even the method described in Patent Document 1 may fail to sufficiently suppress the dissociation of the antibody or the antigen from the carrier particles. Therefore, strategies have been desired by which a favorable measurement accuracy can be maintained even in a case in which dissociation of a substance from an insoluble carrier occurs.

In view of the foregoing, the present disclosure aims to provide an immunoassay reagent that exhibits excellent stability during storage, and an immunoassay method in which the immunoassay reagent is used.

Means for Solving the Invention

The means for solving the foregoing problem includes the following embodiments.

<1> An immunoassay reagent, including:

a substance that exhibits an immune reaction; and
an insoluble carrier having a function to bind to the substance,
the insoluble carrier comprising an insoluble carrier that is bound to the substance and an insoluble carrier that is not bound to the substance.

<2> The immunoassay reagent according to <1>, wherein the substance and the insoluble carrier are bound to each other via a binding pair that consists of a pair of substances having a function to bind to each other.
<3> The immunoassay reagent according to <2>, wherein the binding pair consists of biotin and a biotin-binding protein.

<4> The immunoassay reagent according to any one of <1> to <3>, wherein the insoluble carrier is in a form of particles.

<5> An immunoassay method, including a process of preparing a composition including a substance A and a reagent,

the reagent including a substance B that exhibits an immune reaction with respect to the substance A, and an insoluble carrier having a function to bind to the substance B, and
the insoluble carrier including an insoluble carrier that is bound to the substance B and an insoluble carrier that is not bound to the substance B.

<6> The immunoassay method according to <5>, wherein the composition further includes a substance C that exhibits an immune reaction with respect to the substance A and is different from the substance B, and the substance A is bound to a labeling substance.

<7> The immunoassay method according to <6>, wherein the composition is prepared by conducting a process of mixing the substance A and the substance C, and subsequently conducting a process of mixing a mixture of the substance A and the substance C with the reagent.

<8> The immunoassay method according to <6>, wherein the composition is prepared by conducting a process of mixing the substance C and the reagent, and subsequently conducting a process of mixing a mixture of the substance C and the reagent with the substance A.

<9> The immunoassay method according to <6>, wherein the composition is prepared by conducting a process of mixing the substance A and the reagent, and subsequently conducting a process of mixing a mixture of the substance A and the reagent with the substance C.

<10> The immunoassay method according to <6>, wherein the composition is prepared by mixing the substance A, the reagent, and the substance C, in a single process.

<11> The immunoassay method according to any one of <5> to <10>, wherein the substance B and the insoluble carrier are bound to each other via a binding pair that consists of a pair of substances having a function of binding to each other.

<12> The immunoassay method according to <11>, wherein the binding pair consists of biotin and a biotin-binding protein.

<13> The immunoassay method according to any one of <5> to <12>, wherein the insoluble carrier is in a form of particles.

<14> The immunoassay method according to any one of <5> to <13>, wherein the immunoassay method is electrochemiluminescence immunoassay.

Effect of the Invention

According to the present disclosure, an immunoassay reagent that exhibits excellent stability during storage, and an immunoassay method in which the immunoassay reagent is used, are provided.

Embodiments for Implementing the Invention

In the present disclosure, any numerical range described using the expression "from ... to ..." represents a range in which numerical values described before and after the "to" are included in the range as a minimum value and a maximum value, respectively.

In a numerical range described in a stepwise manner in the present disclosure, an upper limit value or a lower limit value described in one numerical range may be replaced with an upper limit value or a lower limit value in another numerical range described in a stepwise manner. Further, in a numerical range described in the present disclosure, the upper limit value or the lower limit value in the numerical range may be replaced with a value shown in the Examples.

In the present disclosure, in a case in which a respective component in a composition includes plural kinds of substances, the content of the respective component refers to the total content of the plural kinds of substances present in the composition, unless otherwise specified.

<Immunoassay Reagent>

The immunoassay reagent according to the present disclosure (hereinafter, also simply referred to as a reagent) is an immunoassay reagent, including:

a substance that exhibits an immune reaction; and
an insoluble carrier having a function to bind to the substance,

the insoluble carrier including an insoluble carrier that is bound to the substance and an insoluble carrier that is not bound to the substance.

The studies conducted by the present inventors have proved that a favorable measurement accuracy is maintained when the reagent as described above is used in the measurement. The reason for this is thought to be as follows, for example.

The reagent used in the method includes a substance that exhibits an immune reaction, and an insoluble carrier having a function to bind to the substance. Further, the insoluble carrier includes an insoluble carrier that is bound to the substance and an insoluble carrier that is not bound to the substance (hereinafter, also referred to as a dummy carrier).

Namely, the total number of sites, possessed by the insoluble carrier, for binding to the substance that exhibits an immune reaction includes a surplus, and is greater than the total number of sites, possessed by the substance that exhibits an immune reaction, for binding to the insoluble carrier.

Therefore, even if a part of the substance that exhibits an immune reaction dissociates from the insoluble carrier, the dissociated substance is more likely to rebind to the insoluble carrier, as compared with a case in which the insoluble carrier does not include a dummy carrier. As such, it is thought that a favorable measurement accuracy is maintained due to the high likelihood of rebinding.

Further, a part of the substance that exhibits an immune reaction may be immobilized at the insoluble carrier in a manner relatively easy to dissociate, such as physical adsorption, rather than binding to the insoluble carrier. In that case, it is thought that the binding site of the substance that has dissociated from the insoluble carrier is captured by the dummy carrier, and that the amount of the substance remaining free in the specimen without binding to the insoluble carrier is thereby reduced.

The "substance that exhibits an immune reaction" included in the reagent of the present disclosure refers to a substance having an ability to bind to a certain substance as an antigen or an antibody.

The substance to which the substance that exhibits an immune reaction is bound may be a measuring object of the reagent of the present disclosure, or may be a different substance from a measuring object of the reagent of the present disclosure.

Examples of cases in which the substance, to which the substance that exhibits an immune reaction is bound, is a different substance from a measuring object include a case in which the substance that exhibits an immune reaction competes against the substance as a measuring object (i.e., the substance exhibits an immune reaction with respect to a substance, with respect to which the substance as a measuring object exhibits an immune reaction) when the immunoassay is conducted by a competition method.

Examples of the substance that exhibits an immune reaction include an antigen, an antibody, and a synthesized protein including a portion that exhibits an immune reaction.

Examples of the configuration of the insoluble carrier having a function to bind to the substance that exhibits an immune reaction include particles, an immunoplate or a membrane, and the configuration of the insoluble carrier may be selected depending on the method of conducting the immunoassay using the reagent.

From the viewpoint of ease of operation, the insoluble carrier is preferably particles, more preferably particles having magnetic properties (hereinafter, also referred to as magnetic particles).

When the insoluble carrier is in the form of particles, the average particle diameter thereof may be selected from a range of 0.5 $\mu$m to 10 $\mu$m, preferably from 1 $\mu$m to 5 $\mu$m, more preferably from 2.5 $\mu$m to 4 $\mu$m. The foregoing average particle diameter refers to an arithmetic average value of the measured maximum diameters of 50 particles that are randomly selected from those observed with a scanning electron microscope or the like.

When the insoluble carrier is in the form of magnetic particles, the particles may be totally composed of a magnetic body, or the particles may be partly composed of a magnetic body. Examples of the particles that are partly composed of a magnetic body include those having core particles covered with a magnetic layer that includes a magnetic body.

From the viewpoint of reducing the specific weight of the magnetic particles and increasing the dispersibility thereof in a measurement sample, the magnetic particles preferably have core particles including a resin. The type of the resin is not particularly limited, and may be selected from known resins.

The magnetic body included in the magnetic layer may be selected from metals and metal oxides having magnetic properties, such as metals in periods 4-6 and groups 8-10 in the periodic table, lanthanoids and magnetic iron oxides, without particular restriction.

The insoluble carrier has a function to bind to the substance that exhibits an immune reaction.

In the present disclosure, the "function to bind to the substance that exhibits an immune reaction" of the insoluble carrier refers to a function to bind to the substance that exhibits an immune reaction when the insoluble carrier is mixed with the foregoing substance.

It is noted that the foregoing function does not include physical adsorption or the immune reaction of the substance that exhibits an immune reaction included in the reagent.

From the viewpoint of effectively reducing the amount of a free substance in a sample, the foregoing function is preferably a function with a specific property such as immune reaction or biotin-avidin reaction.

The means for providing the insoluble carrier with a function to bind to the substance that exhibits an immune reaction is not particularly limited. From the viewpoint of promoting the rebinding of the substance that has dissociated from the insoluble carrier, the insoluble carrier and the substance that exhibits an immune reaction are preferably bound to each other via a binding pair, i.e., a pair of substances having a function of binding to each other.

The state in which the insoluble carrier and the substance that exhibits an immune reaction are bound to each other via a binding pair may be obtained by, for example, mixing the insoluble carrier to which one of the substances that constitute the binding pair is bound, with the substance that exhibits an immune reaction to which the other one of the substances that constitute the binding pair is bound.

The insoluble carrier having a function to bind to a substance that exhibits an immune reaction but not having the substance bound thereto (dummy carrier) may be obtained by, for example, binding either one of the substances that constitute the binding pair to the insoluble carrier.

From the viewpoint of promoting the rebinding of the substance that has dissociated from the insoluble carrier, the substances that constitute the binding pair are preferably biotin and a biotin-binding protein.

Examples of the biotin-binding protein include streptavidin and avidin, and streptavidin with less non-specific binding is preferred.

The state in which the insoluble carrier and the substance that exhibits an immune reaction are bound to each other via biotin and a biotin-binding protein may be obtained by, for example, mixing the insoluble carrier to which a biotin-binding protein is bound with the substance that exhibits an immune reaction to which biotin is bound; or mixing the insoluble carrier to which biotin is bound with the substance that exhibits an immune reaction to which a biotin-binding protein is bound.

The method for binding the substance that constitutes a binding pair to the insoluble carrier is not particularly limited, and may be performed by a known method.

For example, the substance that constitutes a binding pair may be bound to the insoluble carrier via a functional group disposed at a surface of the insoluble carrier, such as an epoxy group, an amino group, a mercapto group, a carboxy group, or an acid anhydride group.

When the surface of the insoluble carrier includes an inorganic material, for example, the functional group may be disposed at the surface of the insoluble carrier using a silane compound or the like.

The method for binding the substance that constitutes a binding pair to the substance that exhibits an immune reaction is not particularly limited, and may be performed by a known method.

For example, the substance that constitutes a binding pair may be bound to the substance that exhibits an immune reaction by causing a functional group that is bound to the substance that constitutes a binding pair, such as an NHS ester group or a sulfo-NHS ester group, to react with a functional group of the substance that exhibits an immune reaction, such as a primary amino group.

In the insoluble carrier included in the reagent, the proportion of the insoluble carrier that is not bound to the substance that exhibits an immune reaction, with respect to the insoluble carrier that is bound to the substance that exhibits an immune reaction, it not particularly limited.

When the insoluble carrier is in the form of particles, from the viewpoint of increasing the likelihood of rebinding of the substance that has dissociated from the insoluble carrier, the amount of the insoluble carrier that is not bound to the substance that exhibits an immune reaction is preferably 10 parts by mass or more, more preferably 50 parts by mass or more, further preferably 80 parts by mass or more, with respect to 100 parts by mass of the insoluble carrier that is bound to the substance that exhibits an immune reaction.

From the economic viewpoint, the amount of the insoluble carrier that is not bound to the substance that exhibits an immune reaction is preferably 300 parts by mass or less, more preferably 200 parts by mass or less, further preferably 150 parts by mass or less, with respect to 100 parts by mass of the insoluble carrier that is bound to the substance that exhibits an immune reaction.

The reagent of the present disclosure may include a component other than the substance that exhibits an immune reaction, the insoluble carrier, and the binding pair. Examples of the component include proteins (such as BSA), salts, and commercially available additives for immunoassay. Examples of the additive include Blockmaster™ (JSR Corporation).

In the reagent of the present disclosure, the insoluble carrier that is bound to the substance that exhibits an immune reaction and the insoluble carrier that is not bound to the substance that exhibits an immune reaction may be in a mixed state with each other, or may not be in a mixed state with each other (for example, stored in different containers). From the viewpoint of storage stability of the reagent, the insoluble carrier that is bound to the substance that exhibits an immune reaction and the insoluble carrier that is not bound to the substance that exhibits an immune reaction are preferably in a mixed state with each other.

The reagent of the present disclosure may be a combination with a reagent including other components (i.e., a reagent kit). Examples of the reagent include those including a calibrator, a standardized antigen or the like, a sample diluent, a solution for reaction, a BF washing solution, and a luminescent electrolytic solution.

<Immunoassay method>

The immunoassay method of the present disclosure is an immunoassay method, including a process of preparing a composition including a substance A and a reagent,

the reagent including a substance B that exhibits an immune reaction with respect to the substance A, and an insoluble carrier having a function to bind to the substance B, and
the insoluble carrier including an insoluble carrier that is bound to the substance B and an insoluble carrier that is not bound to the substance B.

The reagent used in the foregoing method exhibits a high likelihood of causing the substance B that exhibits an immune reaction with respect to the substance A to rebind to the insoluble carrier, even when the substance B has dissociated from the insoluble carrier. Therefore, a stable measurement precision is maintained according to the method.
In the method of the present disclosure, the substance A may be a substance as a measuring object, or may be a substance that is different from a measuring object.
Examples of a case in which the substance A is different from a measuring object include a case in which the method of the present disclosure is conducted by a competition method, namely, a case in which the measuring object is a substance C that exhibits an immune reaction with respect to the substance A and is different from the substance B. In the method of the present disclosure, a labeling substance may be bound to the substance A.
Specifically, the composition prepared in the method of the present disclosure, including the substance A and the reagent, may be a composition that further includes a substance C, which exhibits an immune reaction with respect to the substance A and is different from the substance B, wherein the substance A is bound to a labeling substance.
For example, when the insoluble carrier, to which the substance B that competes against the substance C is bound, is added to a mixture that includes the substance C as the measuring object and the substance A to which a labeling substance is bound, wherein the concentration of the substance A is known, the substance A that is not reacted with the substance C reacts with the substance B and binds thereto. Therefore, the smaller the amount of the substance C in the mixture with respect to the substance A is, the greater the amount of the substance A that binds to the substance B is and the greater the signal detected from the labeling substance of the substance A is. Based on the forgoing principle, it is possible to calculate the amount of the substance C.
When the measuring object is the substance C that is different from the substance A, the method of preparing the composition that includes the substance A, the substance C and the reagent is not particularly limited. For example, the composition may be prepared by any one of the following four methods.

(Method 1)

Conducting a process of mixing the substance A and the substance C, and subsequently conducting a process of mixing a mixture of the substance A and the substance C with the reagent.

(Method 2)

Conducting a process of mixing the substance C and the reagent, and subsequently conducting a process of mixing a mixture of the substance C and the reagent with the substance A.

(Method 3)

Conducting a process of mixing the substance A and the reagent, and subsequently conducting a process of mixing a mixture of the substance A and the reagent with the substance C.

(Method 4)

Mixing the substance A, the reagent, and the substance C, in a single process.
The reagent used in the method of the present disclosure may be the foregoing reagent of the present disclosure.
Namely, the details and preferred embodiments of the reagent used in the method of the present disclosure may be the same as the details and preferred embodiments of the foregoing reagent of the present disclosure.
In the present disclosure, the labeling substance refers to a substance that engages in the emission of a signal for detection.

The labeling substance may be a substance that emits a signal spontaneously, or may be a substance that emits a signal in an indirect manner.

By binding the labeling substance to the substance A, for example, it is possible to measure the amount of the substance A that is bound to the insoluble carrier via the substance B.

The method for detecting a signal emitted from the labeling substance that is bound to the insoluble carrier is not particularly limited.

Specific examples of the method include electrochemiluminescence immunoassay in which an electrochemiluminescent material is used as a labeling substance (ECLIA), enzyme immunoassay in which an enzyme is used as a labeling substance (EIA), radioimmunoassay in which a radioisotope is used as a labeling substance (RIA), chemiluminescence immunoassay in which a chemiluminescent material is used as a labeling substance (CIA), fluorescence immunoassay in which a fluorescent material is used as a labeling substance (FIA), and bioluminescence immunoassay in which a bioluminescent material is used as a labeling substance (BLIA).

Among these methods, the method according to the present disclosure is preferably conducted by electrochemiluminescence immunoassay (ECLIA).

The method of the present disclosure may include a process of detecting a signal emitted from the labeling substance that is bound to the substance A.

The method for detecting a signal emitted from the labeling substance that is bound to the substance A may be selected depending on the type of the labeling substance.

For example, when an electrochemiluminescent material is used as the labeling substance, the labeling substance is caused to emit light via electrochemical reaction upon application of a voltage thereto, and the intensity of emission is detected.

The type of the labeling substance is not particularly limited, and examples thereof include known labeling substances such as electrochemiluminescent materials, enzymes, radioisotopes, chemiluminescent materials, fluorescent materials and bioluminescent materials.

When an electrochemiluminescent material is used as the labeling substance, specific examples thereof include metal complexes such as ruthenium complexes and osmium complexes.

From the viewpoint of the effect on the immune reaction relevant to the substance A, the molecular weight of the labeling substance is preferably 150,000 or less, more preferably 100,000 or less, further preferably 5,000 or less, particularly preferably 2,000 or less.

Examples of the labeling substance having a molecular weight of 10,000 or less include metal complexes.

The method according to the present disclosure may be applied to the test of a sample obtained from a living body. Specifically, the method may be applied to the detection of a substance included in the sample, or may be applied to the measurement of concentration of a substance included in the sample.

Namely, the substance to be measured by the method of the present disclosure may be in a state of being included in a sample obtained from a living body.

The type of the sample obtained from a living body is not particularly limited, and may be selected from blood, blood serum, blood plasma, lymph fluid, urine, stercus, ascites fluid, pleural effusion, and cerebral spinal fluid.

Examples

In the following, the present disclosure is explained by referring to the Examples. However, the present disclosure is not restricted to the Examples.

As the substance A for a labeling substance to bind to, a mouse-derived monoclonal antibody specifically reactive to human type 1 collagen cross-linked C-terminal telopeptide (anti-1 CTP antibody) was prepared.

As the substance B to be bound to the insoluble carrier, a synthetic peptide including a sequence of 1 CTP and having a biotinylated terminal was used.

As the insoluble carrier having a function to bind to the substance B (hereinafter, also referred to as StAv particles), magnetic particles with streptavidin bound to the surface thereof (Sekisui Medical, average particle diameter: 3 μm) were used.

(Binding of Labeling Substance to Substance A)

The substance A was subjected to solution substitution with PBS-1, and the concentration thereof was adjusted to 2 mg/mL. To the substance A, bis(2,2'-bipyridine)[4'-methyl-4-(4-NHS-4-oxobutyl)-2,2'-bipyridine]ruthenium (II) bis(hexafluorophosphate) (hereinafter, referred to as ruthenium complex NHS, Tokyo Chemical Industry) was added at a molar ratio with respect to the substance A of 1: 20, and allowed to stand at 25°C for 30 minutes. Subsequently, the reaction was terminated by adding a 2M glycine solution by an amount of 6% (w/w) and allowing to stand at 25°C for 30 minutes. The solution after the reaction was applied to Sephadex G-25 equilibrated with 10 mM potassium phosphate/150 mM NaCl (pH 6.0) and the free ruthenium complex was removed. Thereafter, an appropriate fraction was prepared.

The concentration of the substance A to which the labeling substance was bound was calculated by BCA assay (Micro BCA protein assay, Thermo Fisher Scientific). The ruthenium-labeled antibody obtained by the foregoing process was stored under refrigeration.

(Binding of Substance B to StAv particles)

StAv particles (10 mg) were magnetically gathered using a magnet, and a solvent was removed. The StAv particles were washed by adding 1 mL of PBS-1 (140 mM $K_2HPO_4$, 10 mM $KH_2PO_4$, 150 mM NaCl, pH 7.8) and stirring the mixture. The StAv particles were magnetically gathered using a magnet and the medium was removed.

A PBS-1 solution (500 $\mu$L) in which the concentration of the substance B was adjusted to 60 $\mu$g/mL was added to the StAv particles, and the mixture was stirred with a rotor at 25°C for 3 hours. Thereafter, the StAv particles were washed with PBS-1 and PBST (140 mM $K_2HPO_4$, 10 mM $KH_2PO_4$, 150 mM NaCl, 1% Tween 20, pH 7.8). The StAv particles after the washing were subjected to blocking with a solution including an organic polymer agent and BSA, and were stored under refrigeration.

(Preparation of Sample A)

A buffer dispersion, including BSA in which the StAv particles to which the substance B was bound by the foregoing process were dispersed by an amount of 0.5 mg/mL, was prepared as Sample A.

In Sample A, the molar mass of the substance B with respect to the theoretical biotin-binding amount of the StAv particles was 10.

(Preparation of Sample B)

StAv particles to which the substance B was not bound (hereinafter, also referred to as dummy particles) were prepared by a similar process to the foregoing, except that PBS-1 not containing the substance B was used instead of PBS-1 containing the substance B.

The dummy particles were added to Sample A, thereby preparing Sample B, including the StAv particles to which the substance B was bound by an amount of 0.5 mg/mL and the dummy particles by an amount of 0.5 mg/mL, respectively.

(Preparation of Sample C)

Particles to which neither the substance B nor StAv was bound (hereinafter, also referred to as non-binding dummy particles) were prepared by a similar process to the foregoing, except that magnetic particles to which StAv was not bound were used instead of StAv particles.

The non-binding dummy particles were added to Sample A, thereby preparing Sample C, including the StAv particles to which the substance B was bound by an amount of 0.5 mg/mL, and the non-binding dummy particles by an amount of 0.5 mg/mL, respectively.

(Preparation of Sample D)

The substance B was bound to the StAv particles by a similar process to the foregoing, except that the amount of the substance B to bind to the StAv particles was adjusted such that the molar mass of the substance B with respect to the theoretical biotin-binding amount of the StAv particles was 1.

A buffer dispersion, including BSA in which the StAv particles to which the substance B was bound by the foregoing process were dispersed by an amount of 0.5 mg/mL, was prepared as Sample D.

(Preparation of Sample E)

The substance B was bound to the StAv particles by a similar process to the foregoing, except that the amount of the substance B to bind to the StAv particles was adjusted such that the molar mass of the substance B with respect to the theoretical biotin-binding amount of the StAv particles was 0.5.

A buffer dispersion, including BSA in which the StAv particles to which the substance B was bound by the foregoing process were dispersed by an amount of 0.5 mg/mL, was prepared as Sample E.

(Preparation of Sample F)

The substance B was bound to the StAv particles by a similar process to the foregoing, except that the amount of the substance B to bind to the StAv particles was adjusted such that the molar mass of the substance B with respect to the theoretical biotin-binding amount of the StAv particles was 0.2.

A buffer dispersion, including BSA in which the StAv particles to which the substance B was bound by the foregoing process were dispersed by an amount of 0.5 mg/mL, was prepared as Sample F.

<Accelerated Stability Test>

The accelerated stability test was performed to evaluate the storage stability of Samples A to F.

Specifically, Samples A to F were stored at 37°C for a predetermined time, and were subjected to electrochemiluminescence immunoassay (n=3) using the measurement specimens prepared by the following procedures, at the times of before the storage (day 0), one week after the start of the storage (day 7) and three weeks after the start of the storage (day 21), respectively. The results are shown in Table 1A and Table 1B.

(Preparation of Specimens)

As the substance C for the measuring object, a synthetic peptide including 1 CTP sequence was used.

Specimens were prepared by diluting the synthetic peptide including 1 CTP sequence with an antigen diluent (50 mM HEPES, 150 mM NaCl, 10 mM EDTA3Na, 3.0% BSA, 0.01% Tween 20, 0.05% ProClin 300, pH 7.2). The dilution with the antigen diluent was performed in a stepwise manner so as to prepare the specimens used for the respective measurements (calibrator). The antigen diluent was used as the blank specimen. As the test body-like specimen, blood serum obtained from a normal person was spiked with the foregoing peptide, thereby obtaining the specimens at the respective concentrations (panel specimens).

(Electrochemiluminescence immunoassay)

The electrochemiluminescence immunoassay was conducted using a full-automatic electrochemiluminescence immunoassay machine Picolumi III (Sekisui Medical).

First, the specimen (20 $\mu$L) was dispensed into a prescribed reaction tube. Subsequently, the sample contained in a prescribed container was set at a prescribed position of the machine.

The specimen was disposed at a prescribed position of the machine, and the measurement was started. After the start of the measurement, a solution of the ruthenium complex-bound substance A (100 $\mu$L) was added to the reaction tube immediately before being introduced into the machine, and the substance A was caused to react with the substance C in the specimen at 28°C for approximately 8 minutes. Subsequently, the sample (25 $\mu$L) was added to the reaction tube, and the unreacted substance A was caused to react with the substance B bound to the StAv particles at 28°C for approximately 4 minutes.

Thereafter, BF separation was performed with a prescribed BF separator liquid. After the BF separation, the substance A remaining on the StAv particles by the binding to the substance B was suspended in a solution of a prescribed substrate (tripropylamine), and a voltage is applied at a prescribed position of the machine to cause electrochemical luminescence (ECL).

The coefficient of variation of the count values (count CV) was calculated from the values of count obtained by the measurement (n=3) for the calibrator and the panel specimen. The count values at day 7 and day 21 were converted to a relative value with respect to the count value of the calibrator at day 0 as 100%.

The quantitative values of the panel specimen were calculated from the count values obtained by the measurement of the panel specimen, based on the relationship between the theoretical concentration of the calibrator and the count values obtained by the measurement of the calibrator. The quantitative values of the panel specimen at day 7 and day 21 were converted to a relative value with respect to the quantitative values of the panel specimen at day 0 as 100%.

Table 1A

| Count CV | | Sample A | | | Sample B | | | Sample C | | |
|---|---|---|---|---|---|---|---|---|---|---|
| | | day 0 | day 7 | day 21 | day 0 | day 7 | day 21 | day 0 | day 7 | day 21 |
| Calibrator | 0 ng/mL (Initial Count) | 1.2% | 4.4% | 2.7% | 0.2% | 6.2% | 4.4% | 0.4% | 6.7% | 2.7% |
| | 1 ng/mL | 1.3% | 2.1% | 6.3% | 0.7% | 2.2% | 0.7% | 0.2% | 2.2% | 0.9% |
| | 2.5 ng/mL | 0.3% | 4.9% | 12.4% | 1.4% | 2.3% | 1.7% | 0.7% | 1.1% | 1.8% |
| | 5 ng/mL | 1.2% | 3.8% | 4.8% | 0.6% | 1.0% | 1.0% | 0.7% | 1.8% | 0.4% |
| | 10 ng/mL | 2.5% | 10.8% | 1.1% | 0.8% | 6.2% | 3.9% | 1.7% | 1.5% | 5.2% |
| | 25 ng/mL | 1.4% | 6.7% | 5.6% | 2.1% | 0.6% | 0.8% | 0.8% | 7.1% | 4.7% |
| | 50 ng/mL | 1.6% | 2.6% | 2.1% | 1.5% | 4.5% | 7.2% | 2.9% | 6.4% | 1.7% |
| Sp ecimen | panel N | 1.1% | 3.5% | 3.9% | 1.2% | 1.4% | 2.1% | 0.7% | 3.7% | 2.8% |
| | panel L | 0.3% | 2.9% | 6.0% | 1.6% | 2.0% | 1.2% | 2.9% | 2.6% | 1.4% |
| | panel M | 1.6% | 3.0% | 4.6% | 0.7% | 1.2% | 2.2% | 1.5% | 4.5% | 2.2% |
| | panel H | 5.1% | 5.6% | 4.0% | 5.2% | 2.7% | 1.7% | 0.2% | 9.3% | 5.1% |
| | panel UH | 1.3% | 1.7% | 0.5% | 2.4% | 2.9% | 2.5% | 1.0% | 2.7% | 1.6% |
| Count Relative Value (vs day 0) | | Sample A | | | Sample B | | | Sample C | | |
| | | day 0 | day 7 | day 21 | day 0 | day 7 | day 21 | day 0 | day 7 | day 21 |
| Calibrator | 0 ng/mL (Initial Count) | 100% | 60% | 44% | 100% | 89% | 81% | 100% | 81% | 76% |
| | 1 ng/mL | 100% | 57% | 43% | 100% | 90% | 83% | 100% | 86% | 77% |
| | 2.5 ng/mL | 100% | 60% | 48% | 100% | 92% | 80% | 100% | 91% | 78% |
| | 5 ng/mL | 100% | 60% | 44% | 100% | 90% | 78% | 100% | 97% | 88% |
| | 10 ng/mL | 100% | 59% | 47% | 100% | 87% | 77% | 100% | 91% | 82% |
| | 25 ng/mL | 100% | 63% | 51% | 100% | 91% | 78% | 100% | 92% | 83% |
| | 50 ng/mL | 100% | 65% | 52% | 100% | 83% | 71% | 100% | 92% | 84% |
| | Average % | | 61% | 47% | | 89% | 78% | | 90% | 81% |
| Panel Specimen | | Sample A | | | Sample B | | | Sample C | | |
| | | day 0 | day 7 | day 21 | day 0 | day 7 | day 21 | day 0 | day 7 | day 21 |
| Quantitative Value Average | panel N | 3.0 | 2.2 | 2.0 | 2.7 | 2.7 | 2.7 | 2.6 | 5.4 | 4.2 |
| | panel L | 7.0 | 6.5 | 7.2 | 6.6 | 6.7 | 6.2 | 6.1 | 8.9 | 7.4 |
| | panel M | 11.1 | 10.1 | 10.2 | 10.9 | 10.7 | 11.0 | 10.4 | 14.2 | 12.8 |
| | panel H | 29.6 | 25.6 | 28.5 | 29.0 | 27.8 | 30.0 | 27.9 | 31.9 | 30.4 |
| | panel UH | 46.0 | 46.8 | 51.3 | 46.9 | 49.5 | 47.9 | 44.6 | 48.4 | 49.1 |

(continued)

| Panel Specimen | | Sample A | | | Sample B | | | Sample C | | |
|---|---|---|---|---|---|---|---|---|---|---|
| | | day 0 | day 7 | day 21 | day 0 | day 7 | day 21 | day 0 | day 7 | day 21 |
| Relative Value (vs day 0) | panel N | 100% | 71% | 67% | 100% | 100% | 102% | 100% | 205% | 161% |
| | panel L | 100% | 93% | 103% | 100% | 102% | 94% | 100% | 146% | 121% |
| | panel M | 100% | 91% | 92% | 100% | 98% | 101% | 100% | 137% | 123% |
| | panel H | 100% | 87% | 96% | 100% | 96% | 103% | 100% | 114% | 109% |
| | panel UH | 100% | 102% | 112% | 100% | 105% | 102% | 100% | 109% | 110% |
| Quantitative Value CV | panel N | 4.8% | 23.7% | 29.9% | 5.2% | 6.1% | 8.0% | 3.0% | 11.3% | 11.1% |
| | panel L | 0.9% | 7.8% | 15.7% | 3.9% | 4.5% | 2.9% | 8.1% | 5.8% | 3.9% |
| | panel M | 3.3% | 6.0% | 10.3% | 1.3% | 2.1% | 4.4% | 3.4% | 7.8% | 4.4% |
| | panel H | 7.2% | 9.3% | 6.6% | 7.4% | 3.8% | 2.2% | 0.3% | 14.4% | 7.4% |
| | panel UH | 1.6% | 2.9% | 0.9% | 3.1% | 4.1% | 2.6% | 1.2% | 3.9% | 2.0% |

Table 1B

| Count CV | | Sample D | | | Sample E | | | Sample F | | |
|---|---|---|---|---|---|---|---|---|---|---|
| | | day 0 | day 7 | day 21 | day 0 | day 7 | day 21 | day 0 | day 7 | day 21 |
| Calibrator | 0 ng/mL (Initial Count) | 1.4% | 7.0% | 1.1% | 3.6% | 4.0% | 2.4% | 0.6% | 2.0% | 4.2% |
| | 1 ng/mL | 1.3% | 1.9% | 3.9% | 1.7% | 3.3% | 2.6% | 3.9% | 4.3% | 3.0% |
| | 2.5 ng/mL | 1.6% | 5.4% | 3.0% | 1.7% | 2.9% | 3.0% | 1.5% | 3.3% | 2.4% |
| | 5 ng/mL | 2.0% | 10.9% | 4.4% | 2.3% | 6.0% | 2.1% | 3.4% | 2.6% | 2.6% |
| | 10 ng/mL | 3.2% | 5.7% | 7.8% | 2.2% | 3.2% | 4.4% | 5.6% | 6.0% | 0.8% |
| | 25 ng/mL | 1.1% | 3.3% | 2.8% | 1.1% | 2.1% | 2.8% | 0.5% | 2.9% | 15.7% |
| | 50 ng/mL | 0.9% | 3.4% | 2.7% | 1.5% | 5.5% | 1.4% | 3.1% | 1.8% | 4.2% |
| Sp ecimen | panel N | 2.0% | 2.6% | 2.1% | 0.6% | 4.0% | 3.3% | 2.5% | 1.4% | 2.1% |
| | panel L | 0.3% | 0.5% | 1.6% | 1.8% | 5.8% | 6.2% | 4.6% | 2.0% | 5.0% |
| | panel M | 4.7% | 5.1% | 1.4% | 1.4% | 5.6% | 1.7% | 1.4% | 3.5% | 2.2% |
| | panel H | 1.9% | 3.9% | 3.9% | 0.3% | 2.4% | 1.9% | 1.5% | 5.1% | 7.7% |
| | panel UH | 1.2% | 5.5% | 6.0% | 2.5% | 1.5% | 2.9% | 3.4% | 1.1% | 2.5% |

(continued)

| Count Relative Value (vs day 0) | | Sample D | | | Sample E | | | Sample F | | |
|---|---|---|---|---|---|---|---|---|---|---|
| | | day 0 | day 7 | day 21 | day 0 | day 7 | day 21 | day 0 | day 7 | day 21 |
| Calibrator | 0 ng/mL (Initial Count) | 100% | 58% | 43% | 100% | 58% | 42% | 100% | 55% | 41% |
| | 1 ng/mL | 100% | 60% | 45% | 100% | 54% | 40% | 100% | 53% | 40% |
| | 2.5 ng/mL | 100% | 59% | 43% | 100% | 54% | 38% | 100% | 54% | 39% |
| | 5 ng/mL | 100% | 53% | 44% | 100% | 52% | 39% | 100% | 48% | 38% |
| | 10 ng/mL | 100% | 58% | 46% | 100% | 49% | 39% | 100% | 51% | 41% |
| | 25 ng/mL | 100% | 61% | 49% | 100% | 44% | 37% | 100% | 48% | 41% |
| | 50 ng/mL | 100% | 61% | 47% | 100% | 45% | 37% | 100% | 47% | 41% |
| | Average % | - | 59% | 45% | - | 51% | 39% | - | 51% | 40% |

| Panel Specimen | | Sample D | | | Sample E | | | Sample F | | |
|---|---|---|---|---|---|---|---|---|---|---|
| | | day 0 | day 7 | day 21 | day 0 | day 7 | day 21 | day 0 | day 7 | day 21 |
| Quantitative Value Average | panel N | 3.3 | 2.6 | 3.6 | 2.5 | 2.5 | 2.7 | 2.1 | 2.2 | 1.4 |
| | panel L | 7.2 | 6.2 | 7.9 | 6.6 | 6.6 | 6.7 | 6.6 | 5.9 | 6.1 |
| | panel M | 11.0 | 10.1 | 15.3 | 10.5 | 10.5 | 12.9 | 10.9 | 10.6 | 9.8 |
| | panel H | 28.1 | 29.8 | 33.4 | 28.1 | 27.9 | 31.0 | 27.5 | 26.4 | 32.5 |
| | panel UH | 46.6 | 50.0 | 51.5 | 45.8 | 47.9 | 56.3 | 45.6 | 46.2 | 44.8 |
| Relative Value (vs day 0) | panel N | 100% | 79% | 108% | 100% | 98% | 107% | 100% | 104% | 66% |
| | panel L | 100% | 87% | 111% | 100% | 99% | 101% | 100% | 90% | 92% |
| | panel M | 100% | 92% | 139% | 100% | 100% | 123% | 100% | 98% | 90% |
| | panel H | 100% | 106% | 119% | 100% | 99% | 110% | 100% | 96% | 118% |
| | panel UH | 100% | 107% | 110% | 100% | 105% | 123% | 100% | 101% | 98% |
| Quantitative Value CV | panel N | 8.2% | 10.1% | 8.3% | 2.6% | 16.7% | 12.9% | 10.7% | 5.2% | 9.9% |
| | panel L | 0.7% | 1.3% | 4.4% | 4.3% | 11.2% | 13.6% | 8.7% | 3.6% | 10.1% |
| | panel M | 9.0% | 10.8% | 2.9% | 2.6% | 8.4% | 2.8% | 2.0% | 4.9% | 3.6% |
| | panel H | 2.6% | 6.0% | 4.9% | 0.5% | 3.1% | 2.4% | 1.8% | 5.9% | 8.7% |
| | panel UH | 1.6% | 7.2% | 5.3% | 3.1% | 2.2% | 3.0% | 3.7% | 1.3% | 2.5% |

As shown in Table 1A and Table 1B, Sample B, including the StAv particles to which the substance B was bound and the dummy particles, exhibited a suppressed variation in the relative values (vs day 0) of the count, indicating a suppressed decrease in the count during storage at a high temperature environment, as compared with Samples A, D, E and F in which the StAv particles to which the substance B was bound were included without the dummy particles.

Further, Sample B exhibited a smaller value of count CV, indicating a favorable stability.

In the case of Sample C, including the StAv particles to which the substance B was bound and the non-binding dummy particles, while the decrease in the count during storage at a high temperature environment was relatively suppressed, insufficient stability was shown by a significant variation in the relative values (vs day 0) of the quantitative values of the panel specimens. The reason for this is thought to be that, for example, although the substance B can be immobilized at the non-binding dummy particles by way of physical adsorption, it is less likely to exhibit an immune reaction with respect to the substance A as compared with a case in which the substance B is bound to the dummy particles by way of biotin-avidin reaction.

From the comparison of the relative values (vs day 0) of the count between Samples A, D, E and F, with different amounts of the substance B to bind to the StAv particles, it was suggested that the reduction in the amount of the substance B does not suppress a decrease in the count during storage at a high-temperature environment.

<Study on Mechanism of Dummy Particles to Improve Stability>

From the results of the accelerated stability test, contribution of the dummy particles included in Sample B to the improvement in stability was suggested.

Therefore, the following test was conducted in order to study the mechanism of the dummy particles for improving the stability in the count.

(Test 1: <Measurement of Supernatant after Acceleration)

Sample A was stored at 37°C for two weeks. Thereafter, the StAv particles were magnetically gathered and the supernatant was transferred to a new tube. The supernatant in the tube was subjected to centrifugation and the remaining StAv particles were allowed to precipitate. The supernatant after the precipitation of the StAv particles was used as the sample for the electrochemiluminescence immunoassay conducted by the forgoing method (n=3).

The inhibition ratio was calculated by the following formula from the count average value X, in which the measurement was conducted with Sample A, and the count average value Y, in which the measurement was conducted with the antigen diluent.

$$\text{Inhibition ratio (\%)} = (Y-X)/Y \times 100$$

The quantitative value were calculated in the same manner as the accelerated storage test. The results are shown in Table 2.

Table 2

| Sample | Count Average Value | Inhibition Ratio | Quantitative Value |
|---|---|---|---|
| Antigen Diluent | 83530.7 | - | - |
| Sample A | 5733.4 | 93.1% | 94.7 |

As shown in Table 2, the supernatant of Sample A after the storage at 37°C for two weeks included the substance B at a quantifiable amount, indicating the dissociation of the substance B from the StAv particles during the storage at 37°C for two weeks.

(Test 2: Supernatant Substitution after Acceleration)

Sample A was stored at 37°C for two weeks. Thereafter, the StAv particles were magnetically gathered and the supernatant was removed. Subsequently, a solution with the same composition as the removed supernatant was added to the StAv particles by the same amount as the removed supernatant. The resultant was used as the sample for the electrochemiluminescence immunoassay conducted by the forgoing method (n=3). The results are shown in Table 3.

Table 3

| | Sample | Count Average Value | CV | Inhibition Ratio |
|---|---|---|---|---|
| Before Substitution | 0 ng/mL | 27324.9 | 2.7% | - |
| | 1 ng/mL | 26758.4 | 3.9% | 2.1% |
| After Substitution | 0 ng/mL | 56715.2 | 1.5% | - |
| | 1 ng/mL | 50384.0 | 1.7% | 11.2% |

As shown in Table 3, the values of count, CV and inhibition ratio were improved by exchanging the supernatant in the sample after the storage at 37°C for two weeks. The results indicate that the substance B included in the supernatant, which have dissociated from the StAv particles, may affect the measurements.

(Test 3: Effect of Trapping Free Substance by Addition of Dummy Particles)

Two samples were prepared by the method described in the forgoing "binding of the substrate B to the StAv particles", up to the process of mixing the substance B with the StAv particles and stirring the mixture with a rotor at 25°C for three hours.

One of the samples were added with the dummy particles by the same amount of the StAv particles, and the other one of the samples were not added with the dummy particles.

Thereafter, the StAv particles in the samples were magnetically gathered and the supernatant was removed. Subsequently, the samples were added with PBST and stirred with a vortex mixer 10 minutes. Subsequently, the StAv particles in the samples were magnetically gathered and the supernatant was collected as the first washing solution. The foregoing process were conducted for 5 times and the supernatant was collected as the n-th washing solution at the respective processes. The concentration of the substance B (ng/mL) in the washing solution was measured by electrochemiluminescence immunoassay conducted by the forgoing method (n=3). The results are shown in Table 4.

Table 4

|  | Without Dummy Particles | With Dummy Particles |
|---|---|---|
| 1st Washing Solution | 15.4 ng/mL | 9.5 ng/mL |
| 2nd Washing Solution | 5.3 ng/mL | 3.5 ng/mL |
| 3rd Washing Solution | 3.4 ng/mL | 1.9 ng/mL |
| 4th Washing Solution | 2.3 ng/mL | 1.3 ng/mL |
| 5th Washing Solution | 1.9 ng/mL | 1.2 ng/mL |

As shown in Table 4, the samples added with the dummy particles exhibit a lower concentration of the substance B in the respective washing solutions, as compared with the samples not added with the dummy particles. The results suggest that the substance B that has dissociated from the StAv particles has an ability to bind to the dummy particles, and is trapped by the dummy particles.

(Test 4: Possibility of Dummy Particles to Trap Free Substance)

The second washing solution, obtained in the foregoing Test 3, was added with the dummy particles and stirred with a vortex mixer for 1 hour. The resultant was washed with PBS-1, thereby obtaining a sample. For the comparison, a sample in which the same amount of the non-binding dummy particles were added instead of the dummy particles was obtained.

The sample was dispensed to a prescribed reaction tube by an amount of 25 μL, and was subjected to the electrochemiluminescence immunoassay (n=3).

In the assay, only the substance A and the sample were caused to react at 28°C for 4 minutes, unlike the foregoing method. The BF separation and the subsequent processes were performed by the same manner as the foregoing method. The results are shown in Table 5.

Table 5

|  | Sample | Count Average Value | CV |
|---|---|---|---|
| With Dummy Particles | PBST | 174.0 | 6.0% |
| | 2nd Washing Solution | 458086.1 | 0.6% |
| With Non-binding Dummy Particles | PBST | 102.2 | 11.3% |
| | 2nd Washing Solution | 109.1 | 8.0% |

As shown in Table 5, the sample added with the dummy particles exhibits a higher count average value than the sample added with the non-binding dummy particles. The results suggest that the substance B that has been free in the washing solution is trapped at the surface of the magnetic particles, by way of biotin-avidin reaction between the biotin bound to the substance B and the StAv bound to the surface of the magnetic particles.

The disclosure of Japanese Patent Application No. 2022-050801 is incorporated herein by reference in its entirety.

All publications, patent applications, and technical standards mentioned in the present specification are incorporated herein by reference to the same extent as if each individual publication, patent application, or technical standard was specifically and individually indicated to be incorporated by reference.

**Claims**

1. An immunoassay reagent, comprising:

   a substance that exhibits an immune reaction; and
   an insoluble carrier having a function to bind to the substance,
   the insoluble carrier comprising an insoluble carrier that is bound to the substance and an insoluble carrier that is not bound to the substance.

2. The immunoassay reagent according to claim 1, wherein the substance and the insoluble carrier are bound to each other via a binding pair that consists of a pair of substances having a function to bind to each other.

3. The immunoassay reagent according to claim 2, wherein the binding pair consists of biotin and a biotin-binding protein.

4. The immunoassay reagent according to any one of claim 1 to claim 3, wherein the insoluble carrier is in a form of particles.

5. An immunoassay method, comprising a process of preparing a composition comprising a substance A and a reagent,

   the reagent comprising a substance B that exhibits an immune reaction with respect to the substance A, and an insoluble carrier having a function to bind to the substance B, and
   the insoluble carrier comprising an insoluble carrier that is bound to the substance B and an insoluble carrier that is not bound to the substance B.

6. The immunoassay method according to claim 5, wherein the composition further comprises a substance C that exhibits an immune reaction with respect to the substance A and is different from the substance B, and the substance A is bound to a labeling substance.

7. The immunoassay method according to claim 6, wherein the composition is prepared by conducting a process of mixing the substance A and the substance C, and subsequently conducting a process of mixing a mixture of the substance A and the substance C with the reagent.

8. The immunoassay method according to claim 6, wherein the composition is prepared by conducting a process of mixing the substance C and the reagent, and subsequently conducting a process of mixing a mixture of the substance C and the reagent with the substance A.

9. The immunoassay method according to claim 6, wherein the composition is prepared by conducting a process of mixing the substance A and the reagent, and subsequently conducting a process of mixing a mixture of the substance A and the reagent with the substance C.

10. The immunoassay method according to claim 6, wherein the composition is prepared by mixing the substance A, the reagent, and the substance C, in a single process.

11. The immunoassay method according to any one of claim 5 to claim 10, wherein the substance B and the insoluble carrier are bound to each other via a binding pair that consists of a pair of substances having a function of binding to each other.

12. The immunoassay method according to claim 11, wherein the binding pair consists of biotin and a biotin-binding protein.

13. The immunoassay method according to any one of claim 5 to claim 10, wherein the insoluble carrier is in a form of particles.

**14.** The immunoassay method according to any one of claim 5 to claim 10, wherein the immunoassay method is electrochemiluminescence immunoassay.

## INTERNATIONAL SEARCH REPORT

| | International application No. |
|---|---|
| | **PCT/JP2023/012020** |

**A.    CLASSIFICATION OF SUBJECT MATTER**

*G01N 33/531*(2006.01)i
FI:    G01N33/531 Z

According to International Patent Classification (IPC) or to both national classification and IPC

**B.    FIELDS SEARCHED**

Minimum documentation searched (classification system followed by classification symbols)

G01N33/531

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| Y | JP 2000-321279 A (MITSUBISHI CHEMICALS CORP.) 24 November 2000 (2000-11-24) paragraphs [0004], [0005], [0010] | 1-14 |
| Y | JP 2017-533913 A (F. HOFFMANN-LA ROCHE AG) 16 November 2017 (2017-11-16) paragraph [0058] | 1-14 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **01 June 2023** | **13 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
**Information on patent family members**

| International application No. |
| --- |
| **PCT/JP2023/012020** |

| Patent document cited in search report | | | Publication date (day/month/year) | Patent family member(s) | | | Publication date (day/month/year) |
| --- | --- | --- | --- | --- | --- | --- | --- |
| JP | 2000-321279 | A | 24 November 2000 | (Family: none) | | | |
| JP | 2017-533913 | A | 16 November 2017 | US | 2017/0248597 | A1 | |
| | | | | paragraph [0071] | | | |
| | | | | US | 2017/0248598 | A1 | |
| | | | | WO | 2016/071393 | A1 | |
| | | | | WO | 2016/071392 | A1 | |
| | | | | EP | 3018479 | A1 | |
| | | | | EP | 3072902 | A1 | |
| | | | | EP | 3215848 | A1 | |
| | | | | EP | 3215849 | A1 | |
| | | | | CN | 107003309 | A | |
| | | | | CN | 107074921 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000321279 A
- JP 2022050801 A